# EUROPEAN PATENT APPLICATION

(11) **EP 4 710 987 A1**
(43) Date of publication of application: **18.03.2026**
(21) Application number: 24200271.5
(22) Date of filing: 13.09.2024
(51) Int. Cl.: A61M 37/00, A61K 9/00

(54) **MEDICAL IMPLANT FOR RELEASE OF AN AFFINITY-BOUND DRUG IN AN ELECTRIC FIELD**

(71) Applicant: Technische Universität München, in Vertretung des Freistaats Bayern, 80333 München (DE); Medizinische Universität Graz, 8010 Graz (AT)
(72) Inventor: Berensmeier, Sonja, 81829 Munich (DE); Fraga-Garcia, Paula, 85354 Freising (DE); Steegmüller, Tobias, 80337 Munich (DE); Röcker, Dennis, 80805 Munich (DE); Reindl, Marco, 8020 Graz (AT); Schwaminger, Sebastian, 8010 Graz (AT)
(74) Representative: Schiweck Weinzierl Koch Patentanwälte Partnerschaft mbB

(57) **Abstract**

The present invention relates to a medical implant comprising in its interior a device for electrically controllable ad- and desorption of molecules, said device comprising (a) two electrodes comprising a conductive surface, (b) a stationary phase between said two electrodes, wherein said stationary phase is not in direct contact with said electrodes, (c) a controller functionally associated with a power supply configured for applying and changing a voltage between said electrodes, wherein changing the voltage between said electrodes changes the affinity of molecules so as to adsorb said molecules to said stationary phase or desorb molecules from said stationary phase when said molecules are adsorbed to said stationary phase, and the use of said medical implant in the treatment of a medical condition.

## Description

### TECHNICAL FIELD OF THE INVENTION

The present invention relates to a medical implant comprising in its interior a device for electrically controllable ad- and desorption of molecules, said device comprising: (a) two electrodes comprising a conductive surface, (b) a stationary phase between said two electrodes, wherein said stationary phase is not in direct contact with said electrodes, (c) a controller functionally associated with a power supply configured for applying and changing a voltage between said electrodes, wherein changing the voltage between said electrodes changes the affinity of molecules so as to adsorb said molecules to said stationary phase or desorb molecules from said stationary phase when said molecules are adsorbed to said stationary phase. The present invention also relates to a molecule adsorbed to the stationary phase of said implant for use in a treatment of a medical condition with said molecule, said method comprising desorbing said molecule from said stationary phase of said implant implanted into said subject by changing the voltage between said electrodes of said implant. Further provided is a method of treatment of a medical condition of a subject in need of a treatment with a ligand adsorbed to the stationary phase of said implant, comprising desorbing said molecule from said stationary phase of said implant implanted into said subject by changing the voltage between said electrodes of said implant.

### BACKGROUND

Drug delivery systems (DDS) are technological formulations or tools that are used to administer active substances, pharmaceuticals or other substances foreign to the body to achieve a therapeutic effect in humans or animals. In particular, DDS help to achieve therapeutic effects by influencing the absorption, distribution, metabolism and excretion of an active substance at the desired site of action and to minimize possible side effects. Drug delivery technologies have enabled the development of many pharmaceutical products that improve patient health by enhancing the delivery of a therapeutic to its target site, minimizing off-target accumulation and facilitating patient compliance (Vargason et al. 2021, Nature Biomedical Engineering volume 5, 951-967).

Each of the five generations of therapeutics (small molecules, proteins and peptides, antibodies, nucleic acids and cell therapies, such as living cells) have their unique delivery challenges. Regardless of the class of therapeutic, DDS have adopted one or more strategies for drug modification or environmental modification. To give a therapeutic effect, drug molecules need to be released from the DDS and become available for interaction with the body. The selection of a suitable DDS depends on the drug properties, the desired release rate, the desired site of action and the route of administration.

Conventional drug delivery systems such as powders, tablets, capsules, etc., are associated with a variety of drawbacks, severe side effects, multidrug resistance issues, lack of target specificity, etc. In fact, with conventional DDS the drug is diffused throughout the body via systemic blood circulation. For most drugs, only a small portion of them reaches the affected organ, such as in chemotherapy where roughly 99% of the drugs administered do not reach the tumor site.

Due to development of a large number of new drug candidates, improved DDS are being intensively researched and developed to improve pharmacokinetics and pharmacodynamics of drugs and to enable their transport to the target site in the desired dose at the right time and in the right release. However, the development of a novel chemical entity is a very expensive and time-consuming process, and therefore designing of new delivery systems of an already existing drug molecule can significantly enhance drug safety and efficacy.

Lately, nanotechnology-based drug delivery system have been described as a better option to tackle such hurdles and offer many benefits over free drugs. Numerous materials have been used for the development of nanocarriers such as lipids, polymers, metals, etc. Various polymeric nanocarriers, i.e., polymeric micelles, nanocapsules, nanoparticles, nanogels, and dendrimers, have been thoroughly investigated (Hashida 2020, Advanced Drug Delivery Reviews, Vol. 157, 71-82). Drug-loaded nanoparticles can be targeted even more precisely to a specific site of a disease by introducing specific molecules (targeting moieties) on the particle surface. In this respect, nanoparticles have proven to be very suitable for improving the solubility of hydrophobic drugs, biodistribution and pharmacokinetics, and preferential accumulation at the target site (Pircalabioru et al. 2019, Biomedical Applications of Nanoparticles, pages 31-62; Patra et al. 2018, Journal of Nanobiotechnology volume 16, Article number: 71). Both natural and biocompatible synthetic polymers are used to produce polymeric nanoparticles for drug delivery.

However, there are certain limitations surrounding DDS such as multiple reactions with repeated purifications, drug leakage, and low yield. Moreover, active ingredients must be released in a defined concentration in order to avoid damaging healthy cells in cancer therapies, but rather to be able to specifically destroy cancer cells. Thus, the controlled release of active ingredients is necessary for this problem. Current DDS can release active ingredients in a targeted manner, for example through temperature responsiveness. The electrochemical release of active ingredients is also possible, for example via redox-stimuli-responsive polymers (Gu et al. 2018, Coordination Chemistry Review, Vol. 364, 51-85). Further, pH/redox dual responsive nanoparticles have been described to be used for controlled intracellular delivery and tumor-targeting (Feng et al. 2017, Chemistry A European Journal, Vol. 23, Issue 39, 9397-9406). Further, implantable polymeric drug delivery devices, such as reservoirs and monolithic type implants have been described for clinical applications (Stewart et al. 2018, Polymers (Basel), Dec 10(12): 1379). For these implants passive diffusion is the main driver of solute transport.

Hence, there is an ongoing need for developing DDS with better safety and efficacy attributes such as reduced off-target drug action, including side effects and toxicities, and to deliver the drug in the required dose to the target site, and within a specific time, thereby contributing to medication compliance. Thus, there is a constant demand of providing new and sophisticated delivery systems with greater functionality and/or free from known problems and limitations, e.g., as outlined above.

The technical problem therefore is to comply with this need.

### SUMMARY OF THE INVENTION

The technical problem is solved by the subject-matter as defined in the claims.

In some aspects, the present invention relates to a medical implant comprising in its interior a device for electrically controllable ad- and desorption of molecules, said device comprising:
(a) two electrodes comprising a conductive surface,
(b) a stationary phase between said two electrodes, wherein said stationary phase is not in direct contact with said electrodes,
(c) a controller functionally associated with a power supply configured for applying and changing a voltage between said electrodes,
wherein changing the voltage between said electrodes changes the affinity of molecules so as to adsorb said molecules to said stationary phase or desorb molecules from said stationary phase when said molecules are adsorbed to said stationary phase.

It is envisaged that said electrodes of said medical implant form a capacitor. It is further envisaged that said stationary phase acts as a dielectric or insulator between the electrodes. It is also envisaged that each of said electrodes comprises an insulating layer between said electrode and said stationary phase. It is further envisaged that said electrodes are configured to allow flow of a liquid. It is particularly envisaged that said liquid is isotonic with blood. It is also envisaged that said flow of said liquid is in transverse direction and/or in longitudinal direction in relation to said stationary phase being located between said electrodes.

The voltage being changed between said electrodes may be a DC voltage. Said voltage may be from a constant voltage source, preferably an adjustable constant voltage source. It is also envisaged that said controller is a voltage source which does not react on changing current. It is further envisaged that said electrode is not formed by an electrically conductive coating and/or metallic net which is directly formed and/or disposed on said stationary phase.

It is particularly envisaged for said medical implant that said stationary phase is porous. Said stationary phase may be a membrane, a polymer, a molecularly imprinted polymer, a non-metal material, foam or felt. It is further envisaged that said stationary phase comprises at least two stationary phases. It is also envisaged that said at least two stationary phases are stacked, wrapped or tortuous. Further, said at least two stationary phases may be located between two electrodes. It is further envisaged that each stationary phase is located between a separate set of two electrodes. It is also envisaged that said stationary phase is capable of adsorbing said molecules. It is also envisaged that said stationary phase is capable of adsorbing said molecules when the voltage is changed between said electrodes. It is further envisaged that said stationary phase is capable of adsorbing said molecules through a ligand immobilized on said stationary phase.

For said medical implant it is also envisaged that said ligand is capable of non-covalently binding said molecules. Said ligand may be capable of non-covalently binding said molecules when the voltage is changed between said electrodes. It is also envisaged that said stationary phase comprises molecules already adsorbed to said stationary phase or said ligand immobilized on said stationary phase. It is further envisaged that said molecules adsorbed to said stationary phase are desorbed from said stationary phase or said ligands when the voltage is changed between said electrodes.

Said molecules may be biomolecules or chemical molecules. Said biomolecule may be a protein, preferably an antibody or insulin, or a nucleic acid, such as DNA or RNA.

In some aspects the present invention also relates to a molecule adsorbed to the stationary phase of the medical of the present invention for use in a treatment of a medical condition with said molecule, said method comprising desorbing said molecule from said stationary phase of said implant implanted into said subject by changing the voltage between said electrodes of said implant.

In some aspects the present invention also relates to a method of treatment of a medical condition of a subject in need of a treatment with a ligand adsorbed to the stationary phase of the medical implant of the present invention, comprising desorbing said molecule from said stationary phase of said implant implanted into said subject by changing the voltage between said electrodes of said implant.

### BRIEF DESCRIPTION OF THE DRAWINGS

The invention will be better understood with reference to the detailed description when considered in conjunction with the non-limiting examples and the accompanying drawings, respectively.
**Figure 1** shows a possible schematic set-up of the components of the device comprised by the medical implant of the present invention.
**Figure 2** shows the preparation of the stationary phase of the device comprised by the medical implant of the present invention and the adsorption and desorption of molecules to and from said stationary phase.
**Figure 3** shows a schematic illustration of a set-up of the medical implant for controlled drug release.

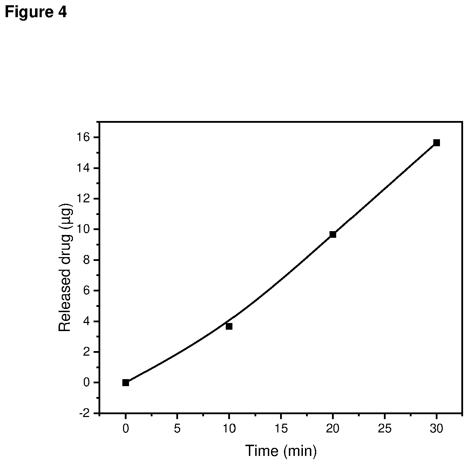

**Figure 4** shows the drug amount released from the medical implant depending on the time in an electric field resulting from a potential of 32 V between two electrodes in a distance of 2 mm.

### DETAILED DESCRIPTION OF THE INVENTION

The present invention is described in detail in the following and is also illustrated by the appended examples and figures.

Controlled drug delivery technologies have progressed over the last six decades (Park 2014, J Control Release. Sep 28; 190: 3-8). Pharmaceutical and biopharmaceutical companies are focusing on R&D to develop new molecules for various therapeutic applications and drug delivery platforms due to its potential implications in the treatment of cancers and other chronic diseases. Lately, nanotechnology-based drug delivery system have been described as suitable option to fulfill the stringent requirements placed on these DDS. A number of drug carriers have been studied to effectively target specific tissues, including liposomes, nanogels, and other nanotechnologies (Patra et al. 2028, J Nanobiotechnology 16: 71; Rao et al. 2028, Design of Nanostructures for Theranostics Applications, Pages 243-275). There are several advantages of using nanotechnology for drug delivery, including precise targeting of specific cells and increased drug potency. Nanoparticles can also carry vaccines to cells that might be hard to reach with traditional delivery methods. However, there are some concerns with the use of nanoparticles for drug delivery. Some studies have shown that nanoparticles may contribute to the development of tumors in other parts of the body. There is also growing concern that nanoparticles may have harmful effects on the environment.

Further, current delivery systems can release active ingredients in a targeted manner, for example through temperature responsiveness of the active agent or other stimuli. Also electrochemical release, for example via redox-responsive polymers, has been described to be used for controlled intracellular delivery and tumor-targeting (Gu et al. 2018, Coordination Chemistry Review, Vol. 364, 51-85; Feng et al. 2017, Chemitry A European Journal, Vol. 23, Issue 39, 9397-9406). Further, implantable polymeric drug delivery devices have been described for clinical applications (Stewart et al. 2018, Polymers (Basel), Dec 10(12): 1379). However, there is an ongoing need for developing DDS for controlled release of active ingredients to a subject. In particular, there is a need of providing DDS with better safety, such as reduced off-target drug action, including side effects and toxicities, thereby delivering the drug in a defined dose to the target site

The present invention takes these necessities into account and relates to novel and improved means that can be used for targeted and localized drug delivery. In particular, the present invention provides for a medical implant for electrically controlled ad- and desorption of molecules from and to a stationary phase inside said implant that can be applied for treating a medical condition or disease in a subject. Thus, the medical implant of the present invention can be used to temporarily or permanently delivering molecules or medicaments to a specific target site. Specifically, the provided medical implant allows for a controlled adsorption and desorption of an active ingredient to and from the stationary phase by utilizing affinity membrane chromatography techniques and applying external potential changes. Thus, by the electrically controlled release of the compound from the implant, the molecules can be delivered in a well controlled and targeted way to specific locations/tissues in the body.

In particular, the present invention makes use of local electric fields to suppress specific electrostatic binding and to break the bonds between the stationary phase and bound molecule so that the molecule is desorbed from the stationary phase and directly delivered to the subject. This has the advantage that the molecule's release from the stationary phase of the implant into the human or animal body can be precisely controlled in terms of time and dose. Moreover, using the implant of the present invention clearly helps to minimize off-target accumulation of active ingredients, i.e. side effects and damage of healthy cells can be significantly reduced. Thus, the surprising advantage when using the implant provided by the present invention is that substances or medicaments comprised in said implant can be released in a precise, electrically controllable way, i.e. gradual or in pulses, thereby permitting the control of the rate, extent and the location of a drug molecule or substance introduced to a subject.

In sum, the DDS technology provided by the present invention allows for a greater functionality and is more convenient for patients in that it helps to facilitate the targeted delivery of drugs in an electrically-induced manner while mitigating their side effects. Therefore, the implant of the present invention can be applied for various medical applications, comprising treatment of acute or chronic conditions or diseases, e.g. cancer.

In some aspects, the present invention relates to a medical implant comprising in its interior a device for electrically controllable ad- and desorption of molecules, said device comprising:
(a) two electrodes comprising a conductive surface,
(b) a stationary phase between said two electrodes, wherein said stationary phase is not in direct contact with said electrodes,
(c) a controller functionally associated with a power supply configured for applying and changing a voltage between said electrodes,
wherein changing the voltage between said electrodes changes the affinity of molecules so as to adsorb said molecules to said stationary phase or desorb molecules from said stationary phase when said molecules are adsorbed to said stationary phase.

As used herein, the term "medical implant" refers to a synthetic product that satisfies functionality demands defined by the working environment, i.e. the human or animal body on or in which the product is implanted. Accordingly, the medical implant of the present invention is configured to be placed inside or on the surface of a subject, without tissue restriction, to deliver medication to a human or animal body. In this respect the medical implant ensures safety and effectiveness to the patient. The medical implant of the present invention may be configured to be placed permanently, i.e. for a certain time period, on or in the subject or can be removed directly after accomplishing the intended function. The "medical implant" of the present invention is not particularly limited in terms of its shape, dimension or material as long as it comprises the device described herein that is configured to allow an electrically controllable ad- and desorption of molecules to and from a stationary phase. In particular, is it envisaged that the medical implant consists of synthetic (bio)materials that are tolerated by the human or animal body.

The medical implant of the present invention comprises in its interior a device for electrically controllable ad- and desorption of molecules to the stationary phase as described elsewhere herein. In particular, said device comprises (a) two electrodes comprising a conductive surface, (b) a stationary phase between the two electrodes, wherein said stationary phase is not in direct contact with said electrodes, and (c) a controller functionally associated with a power supply configured for applying and changing a voltage between said electrodes.

The term "electrically controllable" as used herein may refer the ability of the implant to manipulated, adjusted, and/or regulated the ad- and desorption of molecules to and from the stationary phase using an electric signal or voltage. In particular, the medical implant of the present invention is based on "electrically controllable" affinity chromatography using "electrochemically-modified" means and methods relating to affinity chromatography, preferably Liquid Affinity Chromatography. Thus, the implant of the present invention specifically utilizes "digital" (i.e. "electrically controllable" and/or "electrochemically-modified") affinity chromatography for a electrically controlled ad- and desorbing molecules to and from a stationary phase in a targeted manner.

The term "affinity chromatography" as used herein may refer to a type of liquid chromatography that for example uses a biologically-related agent (e.g., chemical entity or biomolecule, e.g., immobilized chemical entity or biomolecule) as a stationary phase to specifically bind the molecule(s) of interest. Affinity chromatography exploits the specific interaction between two molecules or chemical entities, for example, an enzyme and its substrate, an antibody and its antigen, or a receptor and its ligand. The term "liquid chromatography" as used herein may refer to a separation technique used, for example, in analytical chemistry and biochemistry, to separate and/or analyze components of a mixture based on their interactions with a liquid mobile phase and a stationary phase.

"Adsorption" means that that molecules, e.g. initially dissolved in a liquid, are bound/adsorbed to the stationary phase. "Desorption" means that said molecules are again released/desorbed from said the stationary phase and e.g. return into a liquid. Specifically, changing the voltage between the electrodes comprised by the device comprised by the medical implant of the present invention can change the affinity of the molecules so that to adsorb said molecules to the stationary phase. Similarly, changing the voltage between the electrodes comprised by the device comprised by the medical implant of the present invention can also changes the affinity of the molecules so that to desorb said molecules from the stationary phase. "Changing the affinity" means that the affinity between the molecule and the stationary phase is modified.

The term "affinity" may describe a natural attraction or force between entities or substances that causes them to combine or interact. When referring to "affinity of chemical entities," especially in a biochemical or molecular context, it may denote the measure of the attraction or binding strength between two molecules or chemical entities, such as a ligand and its receptor, an enzyme and its substrate, or an antibody and its antigen. This affinity is crucial for determining the specificity and efficacy of biological interactions and can be influenced by various factors, including the molecular structure, charge, and environmental conditions. The higher the affinity, the stronger the attraction or binding force between the two entities. Thus, in the context of the present invention, "changing the affinity" means that the binding strength of the molecules to the stationary phase is changed by changing the voltage between the two electrodes so that the molecules adsorb to or desorb from the stationary phase.

For example, a first change in the voltage between said electrodes comprised by the device of the medical implant of the present invention may change the affinity of said molecules to said stationary phase so that the affinity to said stationary phase is increased, i.e. the increased affinity leads to an increased binding of said molecules to said stationary phase. Accordingly, said molecules are e.g. desorbed from a liquid and adsorbed to said stationary phase. Then, the implant is brought on the surface of or inside a human or animal body and a further change in the voltage between said electrodes may again decrease the affinity of said molecules to said stationary phase so that the molecules are desorbed from said stationary phase and adsorbed into the liquid. Thus, the further voltage change allows for a controlled release of the molecules from the medical implant, thereby delivering the molecules to the subject. Accordingly, the (bio)molecule's adsorption to and desorption from the stationary phase is controlled by voltage changes. Thus, by applying an electric potential, binding and release of the (bio)molecules to and from the stationary phase can be controlled by electrical charges.

Hence, once the implant is brought on the surface of or inside a human or animal body, further voltage changes, e.g. pulses, and/or continuous voltages increases can be applied so as to desorb the molecules from the stationary phase in a controlled manner. This allows for a gradual release to the subject as described elsewhere herein.

In particular, the medical implant of the present invention is configured to allow a electrically controllable ad- and desorption of molecules to and from a stationary phase by means of (liquid) affinity chromatography. Specifically, the medical implant is configured to comprise a device comprising two electrodes comprising a conductive surface, a stationary phase between said two electrodes, wherein said stationary phase is not in direct contact with said electrodes, and a controller functionally associated with a power supply configured to apply and change a voltage between said electrodes. In this respect changing the voltage between said electrodes changes the affinity of the molecules so as to adsorb said molecules to said stationary phase or desorb said molecules from said stationary phase.

The term "voltage" often denoted as V as used herein may refer to the measure of electrical potential difference between two points in an electrical circuit. It represents the energy (per unit charge) required to move an electric charge between those two points. The unit of voltage is the volt (V), which is defined in the International System of Units (SI) as one joule per coulomb. In some embodiments the voltage is a "direct current voltage", often abbreviated as "DC voltage". "DC voltage" may refer to a type of electrical voltage that remains constant in polarity and magnitude over time. In some embodiments the voltage can be from a constant voltage source, preferably an adjustable constant voltage source. The term "constant voltage source" as used herein may refer to an electrical device or circuit that generates a steady and unchanging voltage output, regardless of changes in the connected load or other external factors. The term "adjustable constant voltage source" as used herein may refer to an electrical device or circuit that provides a stable and unchanging voltage output that can be adjusted (e.g., manually) to different levels. "Changing the voltage" means that the voltage between the two electrodes is alter, e.g. decreased or increased, which leads to a variation in the flow of the electric charge over time in an electrical circuit, specifically a variation in the flow of the electric charge between the two electrodes. Thus, the term "changing the voltage between said electrodes" as used herein may refer to the adjustment or modification of the electrical potential difference between two specific electrodes in a given setup or device comprised by the medical implant of the present invention.

Preferably, the voltage applied is in the range from -100 V to + 100 V, more preferably from -50 V to + 50 V. Stronger potentials may lead to oxidation of the (bio)molecules as well as the formation of hydrogen, which might endanger the system.

In the context of the present invention, the voltage between the two electrodes is changed by a controller functionally associated with a power supply configured for applying and changing the voltage between said electrodes. In some embodiments said controller is a voltage source which does not react on changing current. The term "changing current" as used herein may refer to a fluctuation or variation in the flow of electric charge (current) over time in an electrical circuit.

In further aspects, by elevating the voltage applied to a specific level, repulsive forces can be generated, particularly through the electric field brought forth by the applied voltage. This electric field has the potential to alter the overall interaction force between the biomolecules and the stationary phase, especially the binding sites, transitioning it from attractive to repulsive. These effects are contingent upon minor shifts in the biomolecules' conformations, specifically secondary, tertiary, or quaternary structures (e.g., in proteins). These shifts result in the loss of complementary features between at least one biomolecule and/or binding site due to their interaction with the induced or established electric field from the applied voltage. In further aspects, these subtle conformational changes generally do not lead to denaturation, aggregation, or similar modifications of the (bio)molecules. Additionally, these conformational changes tend to be transient, existing only temporarily and being easily reversed. This reversal typically occurs automatically as the biomolecules move away from the influence of the electromagnetic or electric field and are introduced into the body.

It is also envisaged that the voltage is elevated only for a short time, i.e. in form of a pulse, thereby leading to a controlled release of a portion of the molecules adsorbed to the stationary phase. Then, further pulses can follow resulting in a timely controlled release of the remaining molecules from the stationary phase over a certain period of time. Similarly, the voltage can be increased gradually to achieve an increasing release of the molecules from the stationary phase, thereby permitting control of the rate and extent of the drug molecule or substance introduced to a subject. Thus, suitable adjustments of the release rate may be made in view of the respective molecules desorbed to the stationary phase and the desired result of the treatment/application. Accordingly, the medical implant of the present invention can be used to deliver molecules or medical drugs over longer time periods and in appropriate pharmaceutical doses to a subject.

The term "electrodes" as used herein may refer to conductive materials that are used to establish an electrical connection with a nonmetallic part of a circuit. Essentially, they may act as the interface between electrical circuits and ionic conductors (like electrolytes in a solution). There are generally two electrodes in an electrochemical cell: Anode, which is the electrode where oxidation (loss of electrons) occurs. In a galvanic or voltaic cell (like batteries), it is the negative electrode. However, in an electrolytic cell (used for electrolysis), it is the positive electrode. Cathode, which is the electrode where reduction (gain of electrons) occurs. In a galvanic or voltaic cell, it's the positive electrode. In an electrolytic cell, it's the negative electrode. The choice of electrode material may be crucial and can vary based on the application. Some common electrode materials include metals like platinum, gold, and silver, as well as carbon in various forms (e.g., graphite rods or carbon paste).

According to the medical implant of the present invention, said electrodes are envisaged to be configured to allow flow of a liquid. The term "electrode is configured to allow flow of a liquid" as used herein may indicate that the electrode has been designed or set up in a manner that permits the movement or passage of a liquid. In this context, the electrode's structure or arrangement is such that a solution or liquid as defined elsewhere herein can move across or through it. Further, the electrode is arranged and configured to generate an electric field at the stationary phase.

In some embodiments the electrode is positioned proximal to the volume of the device. As used herein, the expression "proximal to the volume" indicates that the element concerned, i.e. the electrode, is disposed within a sufficiently close distance to the volume so that a electric field sufficient for using the implant of the present invention, i.e. adsorbing and desorbing the (bio)molecules at the stationary phase, may be established. Therefore, this expression encompasses a configuration where the element concerned is not in direct contact with, but is adjacent to, the volume and also a configuration where the element concerned is in direct contact with the volume. For example, the electrode may be disposed inside the device. In such a scenario, the electrode is in direct contact with the volume of the device.

It is particularly envisaged that said electrodes comprised by the device form a capacitor. The term "capacitor" as used herein may refer to an electronic component designed to store and release electrical energy in an electrical circuit. For example, it may consist of two conductive plates separated by an insulating material called a dielectric as described elsewhere herein. The electrodes or conductive plates are made of conductive material that allows electric current to flow through it. Conductive materials have a high density of electric charge carriers (usually electrons) that are free to move, facilitating the transfer of electric charge from one point to another. Conductive surfaces are characterized by their low electrical resistance. Metals, such as copper and aluminum, are some of the most common conductive materials due to their abundance of free electrons that can move easily within their crystalline structures. However, other materials, like certain types of carbon, conductive polymers, and even some liquids and solutions, can also exhibit conductive properties under specific conditions.

In the context of the present invention one or more of the electrodes, including possibly all of them, may be created using a conductive material, particularly a metal coating. The term "electrically conductive coating" may refer to a thin layer or film of material that is applied onto a surface to enhance its electrical conductivity. This coating is designed to allow the passage of electric current across the surface of the electrodes, enabling efficient transfer of electrical charge. The coatings may contain conductive particles or additives, such as metal powders or carbon-based materials, that create pathways for electric charge to flow.

The term "stationary phase" as used herein indicates a physical entity which may be brought into contact with the molecules to be delivered to a subject, e.g. with a liquid comprising the biomolecules, and on which the biomolecules adsorb based on voltage changes as described elsewhere herein. Thus, the "stationary phase" may refer to the "immobile phase" or substance that retains the substance or medicament that needs to be introduced on or into a subject. As liquid passes through and/or over this stationary phase, the compounds or molecules (e.g., chemical entities) interact with the stationary phase in a electrically controllable way, i.e. by changing the voltage, leading to the adsorption to the stationary phase. Thus, the stationary phase is capable of adsorbing said molecules. Specifically, the stationary phase is capable of adsorbing said molecules when the voltage is changed between said electrodes. Thus, the stationary phase is the area or space inside the device comprised by the implant where the molecules or substances will be collected or concentrated and from which the biomolecules will be subsequently released when the implant is used for desorbing the biomolecules from said stationary phase during a certain medical treatment.

The medical device of the present invention may be implemented in various tissues and organs of the subject, i.e. in different physiological environments. Accordingly, the stationary phase is not limited to any particular size, shape or material as long as the functionalities described herein may be satisfied on the surface or inside the subject.

In the context of the present invention the stationary phase is not in direct contact with the electrodes. The term "not in direct contact" as used herein means that that the stationary phase and the two electrodes do not touch each other. Rather there is a certain space or distance between the stationary phase and the two electrodes. In particular, it is envisaged within the context of the present invention that said electrodes are not formed by an electrically conductive coating and/or metallic net which is directly formed and/or disposed on said stationary phase.

It is further envisaged that the stationary phase can act as a dielectric or insulator between the electrodes. The term "dielectric" as used herein may refer to a material that can store and transmit electrical energy in the form of an electric field without allowing significant electric current to flow through it. Dielectrics are insulating materials that possess the ability to polarize when subjected to an external electric field. This polarization involves the displacement of electric charges within the material, resulting in the buildup of positive and negative charges on opposite sides. Dielectrics are characterized by their electrical properties, including their ability to resist the flow of electric current (high electrical resistance) while still allowing the transmission of electric fields. The term "insulator" as used herein may refer to a material that does not allow the easy flow of electric current through it. It possesses high electrical resistance, meaning that it restricts the movement of electric charges, such as electrons. Insulators are characterized by their ability to resist the passage of electricity and prevent the transfer of electrical energy. Common examples of insulating materials include rubber, plastic, glass, ceramic, and most non-metallic materials.

As explained above, the nature and composition of the stationary phase may vary depending on the type of implant and the intended application. However, the stationary phase typically remains fixed in its position inside the implant, contrasting with the mobile phase that moves or flows through and/or over it. Suitable stationary phases that can be applied in the context of the present invention are known in the art and can e.g. be derived from any suitable porous substrate(s), such as membranes, specifically polymeric membranes, serving as the foundational material for the stationary phase. Suitable stationary phases applicable in the context of the present invention include, but are not limited to, stationary phases made from a membrane, a polymer, a molecularly imprinted polymer (MIP), a non-metal material, foam or felt. Preferably the stationary phase is made from polymers or molecularly imprinted polymers. Preferably, the stationary phase is characterized by porosity and/or permeability. The terms "Porosity" or "porous" as used herein mean that the stationary phase consists of a solid matrix with defined holes or pores which have diameters ranging from less than 2 nm to more than 20 µm. The term "permeability" refers to the ability of the stationary phase to transmit fluids. The separation of solutes by porous membranes is mainly a function of molecular size and membrane pore size distribution. In particular, the stationary phase is permeable, in particular to fluids, e.g., water or buffer. Thus, the stationary phase preferably allows the passage of fluids.

However, in the context of the present invention it is particularly envisaged that said electrode is not formed by an electrically conductive coating and/or metal net which is directly formed and/or disposed on the stationary phase. That means, in the context of the present invention the electrode is not created or composed of a conductive material that is directly applied to the surface of the stationary phase, or the way that the electrodes are present in the stationary phase. Instead, as described elsewhere herein, the stationary phase is not in direct contact with said electrodes.

In the context of the present invention, at least one of the stationary phases, possibly a membrane, or a non-conductive carrier may function as an insulating barrier between the electrodes. To achieve this, materials like gold or platinum can be used for constructing the electrodes or the conductive coatings. These metals can be used in their pure form, and the coatings consist solely of a single metal. In some aspects, the stationary phase, particularly a membrane, itself lacks conductivity, relying on the electrical conductivity exclusively from the conductive coating. In particular, the approach involves the use of only one specific type of conductive material or metal for both the electrodes and conductive coatings.

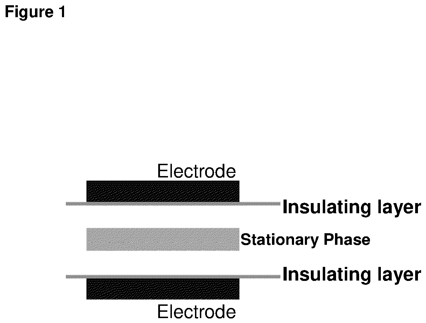

In the context of the present invention it is further particularly envisaged that each of said electrodes comprises an insulating layer between said electrode and said stationary phase as shown in Fig. 1. Thus, it is envisaged in the context of the present invention that the stationary phase and the electrodes do not directly interact. The term "insulating layer between said electrode and said stationary phase" as used herein may refer to a non-conductive barrier or material positioned between an electrode and a stationary phase (e.g. in a chromatographic system). The "insulating layer" prevents direct electrical contact between the electrode and the stationary phase, maintaining their separation and preventing unwanted interactions. Non-limiting examples of the insulating layer can be made from materials also described herein for the stationary phase when acting as "insulator", i.e. materials possessing high electrical resistance that do not allow the easy flow of electric current through them. In particular, the "insulating layer" may be made of non-metallic materials like rubber, plastic, glass, ceramic, coatings with high electrical resistance or inorganic materials like oxides. Preferably, the "insulating layer" may be made of non-conducting polymers, such as electropolymerized non-conducting polymers.

It is further envisaged that the medical implant of the present invention may have a housing into which the implant is placed. Materials applicable for the housing are e.g. polymers, such as silicone, polyurethane, polyethylene, polypropylene, polyterafluoroethylen, polyacrylic acid, polyhydroxyalkaloates, polydimethoxysilane, polyamide, and polyethylentherephthalate.

Accordingly, the housing can determine the shape of the medical implant as described elsewhere herein. Moreover, the housing may help to safely hold the implant in a stable position to allow the intended ad- and desorption of molecules to and from the stationary phase. Thus, the implant may be fixed in said housing e.g. by suspensions or is embedded in this housing. In some embodiments the housing may comprise more than one implant, such as 2, 3, 4, 5, 6, 7, 8, 9, 10 or more implants that are hold or fixed inside said housing and are used in parallel or consecutively for the intended application or treatment. In the context of the present invention it is further envisaged that the housing of the implant is permeable. "Permeable " means in this respect that the housing can be penetrated or passed through by the molecules and liquids described elsewhere herein.

It is further envisaged that the medical implant of the present invention has at least one inlet that is configured to allow introduction of the molecule(s) into the implant so that the molecule(s) can adsorb to said stationary phase. It is further envisaged that the medical implant has at least one outlet that is configured to allow release of the molecule(s) from the implant after desorption from the stationary phase. The inlet and the outlet are configured to allow an electrically controllable ad- and desorption of molecules to and from a stationary phase comprised in said implant. Preferably, the inlet and the outlet are configured to allow a flow of a liquid comprising the molecule(s) into the implant and out from the implant. Suitable liquids are described elsewhere herein. Thus, the molecule(s) described herein may be introduced into the implant via a liquid and by changing the voltage the molecule is then adsorbed to the stationary phase comprised by the device in the interior of the implant. Similarly, the molecule(s) described herein may be desorbed from the stationary phase by changing the voltage and are then released from the implant via a liquid. In some embodiments the medical implant can have more than one inlet and one outlet, such as 2, 3, 4, 5, 6, 7, 8, 9, 10 or even more inlets and outlets, wherein each of said inlets is configured to allow introduction of the molecule(s) into the implant and each of said outlets is configured to allow release of the molecule(s) from the implant.

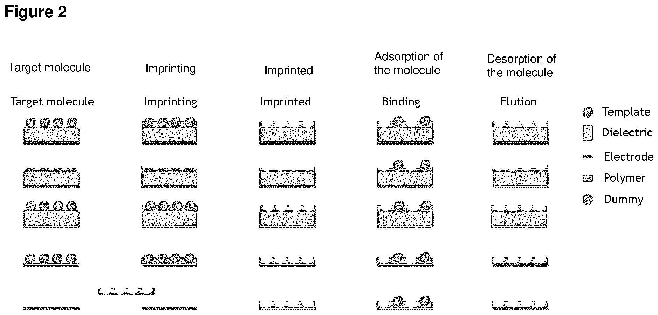

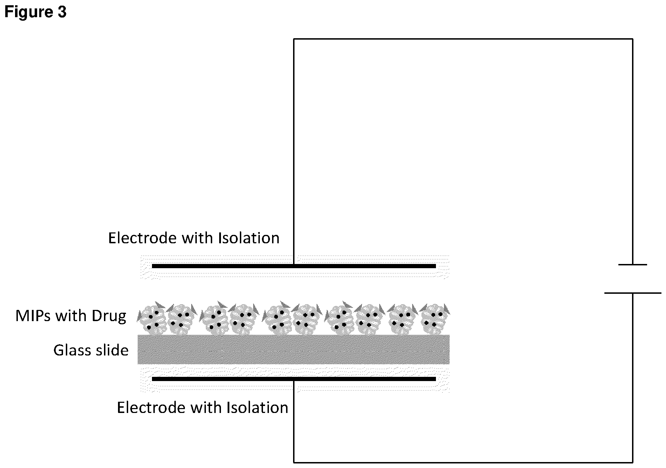

However, in the context of the present invention it is also envisaged that the molecule(s) may be adsorbed to the stationary phase before the stationary phase is positioned between the two electrodes of the device in the interior of the implant. In this case the molecule(s) may not be introduced into the implant via an inlet. Rather, the molecule(s) may be adsorbed/bound to the stationary phase as shown in **Figures 2** **and** **3****,** i.e. prior to placing the stationary phase into the device comprised by device of the implant. In this respect, the stationary phase may be pre-loaded with said molecule(s) outside the implant. Nonetheless, also here the molecule(s) may be released from the implant via the outlet or the outlets once the implant has been placed on or in a subject.

Thus, as described herein above, in the context of the present invention it is also envisaged that said stationary phase comprises molecules already adsorbed to said stationary phase or molecules already adsorbed to ligands immobilized on said stationary phase as described elsewhere herein. Thus, the stationary phase between the two electrodes comprised by the implant of the present invention may already comprise the molecules or chemical entities that shall be desorbed from said stationary phase by changing the voltage between the electrodes as described elsewhere herein. Accordingly, in some embodiments the medical implant comprises a stationary phase already comprising (bio)molecules immobilized on the chosen stationary phase or bond to a ligand immobilized on the stationary phase which is placed between the two electrodes inside the device of the implant of the present invention. Then, applying a voltage change between the two electrodes, thereby changing the affinity between said (bio)molecules and said stationary phase or said ligand immobilized to said stationary phase leads to a decreased binding affinity of said (bio)molecules to said stationary phase or said ligand immobilized to said stationary phase, thereby desorbing the (bio)molecules from the stationary phase and releasing the (bio)molecules into a liquid, thereby introducing the (bio)molecules to the subject.

In the context of the medical implant of the present invention it is also envisaged that the stationary phase may be separated from a (first) liquid used to adsorb said (bio)molecules to said stationary phase and is then positioned between the two electrodes of the device in the interior of the implant. "Separated" means in this respect that the liquid is partly or completely removed from the implant. Then, once the implant is placed on or in a subject, a further liquid, such as a body fluid, will enter the implant and may then be present inside the implant when desorbing the (bio)molecules from the stationary phase.

In some embodiment the medical implant of the present invention is a microchip, which allows a precise release of molecules adsorbed to the stationary phase in said implant in a small scale (e.g. sub-millimeter to millimeter). Preferably, the implant comprising in its interior the device described elsewhere herein has a size ranging from 1 µm³ to 1,000 mm³, more preferably 1 µm³ to 100 mm³, and even more preferably 1 µm³ to 10 mm³. Thus, the implant may be configured for microscopic treatment. However, the medical implant of the present invention may also have a structure to be used for the treatment of a much larger scale. Accordingly, the implant comprising in its interior the device described elsewhere may also have a size ranging from 1µm to 10cm. Thus, also larger implants in the cm range with respect to one, two or all three dimensions of the implant are envisaged.

The implant of the present invention may also comprise additional functional units. In some embodiments, the implant may further comprises or is connected with a supply unit or storage unit that is in fluid communication with the inlet of the implant for supplying the liquid comprising the molecule(s) to be adsorbed to the stationary phase. In this respect the supply unit may be a reservoir for the molecules or substances to be delivered to the subject via the medical implant. Preferably, said molecules are maintained in a liquid inside said supply unit and will then be introduced into the implant, e.g. by means of a syringe or a pump. Further, the molecules may be pre-treated in said supply unit if this is deemed necessary in some embodiments.

In the context of the present invention, the electrodes comprised in the device of the medical implant are configured to allow flow of a liquid. Thus, inside the device there may be a fluid path for the liquid and the volume for containing the liquid along said fluid path may be configured in a manner that the liquid is at least temporarily held within the volume of the chamber to allow adsorption of the molecules to the stationary phase. Preferably, the volume for containing the liquid along said fluid path coincides with the inner volume of the device. However, the volume for containing the liquid along said fluid path may also be smaller than and encompassed by the inner volume of the device. It is preferred that the volume for containing the liquid along said fluid path covers a full cross section of the inner volume of the device along substantially the length of the stationary phase. Preferably, said flow is a continuous flow, i.e. the liquid flows continuously trough said implant.

Thus, the implant may allow that the liquid travels through the device, i.e. the molecules desorbed from the stationary phase flow through the device in an electrically controlled manner. The liquid may also be referred to as mobile phase that passes the fluid path between the electrodes. The term "mobile phase" as used herein may refer to a phase that retains the molecules or substances to be introduced to a subject. The mobile phase thereby moves in a definite direction in the implant in order to release the molecule(s) from said implant into the body, preferably via the outlet(s).

In some embodiments, the fluid path is in traverse direction and/or in longitudinal direction in relation to said stationary phase being located between said electrodes. The term "transverse direction" as used herein may refer to a perpendicular orientation or movement relative to a specified reference point or axis. The term "longitudinal direction" as used herein may refer to the primary or main orientation or movement along a specified reference point or axis.

The fluid used to introduce the molecules into the implant is preferably selected to retain the (bio)molecules or substances in their native state. Preferably, the fluid may be a suitable aqueous solution or buffer as defined elsewhere herein. Thus, the liquid used to introduce the molecule(s) into the implant so that they can adsorb to the stationary phase may be a liquid media or buffer in which biomolecules such as proteins and nucleic acids are stored or stabled. The term "buffer" as used herein may refer to a solution that resists significant changes in pH when small amounts of an acid or a base are added to it. A buffer system generally contains a weak acid and its conjugate base or a weak base and its conjugate acid. For example, a common buffer system is made of acetic acid (a weak acid) and its conjugate base, the acetate ion. The pH range over which a buffer effectively resists changes in pH is typically close to the pKa of the weak acid or base used in the buffer system. The Henderson-Hasselbalch equation is often used to calculate the pH of a buffer solution, given the concentrations of the weak acid and its conjugate base (or vice versa).

Typical buffers that may be used as liquids in the context of the implant of the present invention comprise phosphate-buffered saline (PBS) or Tris-buffered saline (TBS). Thus, the liquid that may be used to introduce the molecules into the implant and to adsorb to the stationary phase outside the body may be characterized by lower ion/salt concentrations, i.e. below 150 mM. PBS or similar solutions used for rinsing, as second fluids, or at any stage following binding and preceding elution, may exhibit salt concentrations approximating or below 150 mM. However, it is preferable to use fluids (rinsing fluids, second fluids, or any fluid) with even lower salt concentrations, such as those typically below 10 mM, particularly below 5 mM, and more specifically below 2 mM, 1 mM, 0.5 mM, and preferably even below 0.25 mM, with an approximate value of 0.15 mM being most favored. As a result, the addition of salt solutions with concentrations exceeding the common buffer concentrations described above is generally avoided when adsorbing the molecules to the stationary phase.

However, the implant provided by the present invention is also configured to allow for the use of fluids comprising higher salt concentrations, such liquids that are isotonic with blood. In some embodiments it is envisaged that said salts have a concentration of more than 0.15 mM, 0.25 mM, 0.5 mM, 1 mM, 2 mM, 5 mM, 10 mM or 150 mM.

When delivering the molecule(s) from the medical implant to a target region of a subject, the medical implant is preferably used with a liquid that is isotonic to blood. "isotonic to blood" means in this respect that the implant can be used in or with a liquid characterized by osmolality and ion concentrations that mirror those found in the human body. Said liquids may have a salt solution of about 150mM or more.

With the means provided by the present invention, it is also possible that the same type of liquid (i.e. having the same salt concentration and the same pH) can be used throughout the whole electrically controlled adsorption and desorption process described herein, i.e. for the adsorption of the molecules to the stationary phase and the desorption of the molecules from the stationary phase. However, as explained above, within the means provided by the present invention it is also possible to use another liquid for the adsorption of molecules to the stationary phase and the desorption from the stationary phase so as to deliver the molecules to the subject. Thus, a specific liquid may be used for the adsorption and desorption step.

The molecules adsorbed to and desorbed from the stationary phase of the medical implant, sometimes also named "substances" or "active ingredients" within the context of the present invention, are preferably biomolecules or chemical molecules. Said molecules may relate to different types of molecules or substances or mixtures thereof that are to be introduced into a subject for therapeutic purposes.

The term "biomolecule" as used herein may refer to a biological molecule, which is any molecule that is involved in the structure, function, and/or regulation of living organisms. Biomolecules comprise a wide range of molecules found within cells and organisms, including macromolecules such as nucleic acids (DNA and RNA), proteins, antibodies, but also carbohydrates, and lipids, as well as smaller molecules like metabolites, cofactors, vitamins, hormones and signaling molecules. In the context of the present invention, these biomolecules, as mentioned above, are each capable of binding to the stationary phase described elsewhere herein. "Capable of binding" means that each of said (bio)molecules is capable of interacting with the surface of said stationary phase, thereby at least temporarily being immobilized on the stationary phase.

The term "protein" is equally used herein with the term "polypeptide". Proteins (including fragments thereof, preferably biologically active fragments, and peptides, usually having less than 30 amino acids) comprise one or more amino acids coupled to each other via a covalent peptide bond (resulting in a chain of amino acids). The term "polypeptide" as used herein describes a group of molecules, which, for example, consist of more than 30 amino acids. Polypeptides may further form multimers such as dimers, trimers and higher oligomers, i.e. consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc. An example for a heteromultimer is an antibody molecule, which, in its naturally occurring form, consists of two identical light polypeptide chains and two identical heavy polypeptide chains. The terms "polypeptide" and "protein" also refer to naturally modified polypeptides/proteins wherein the modification is effected e.g. by post-translational modifications like glycosylation, acetylation, phosphorylation and the like. Such modifications are well known in the art.

The term "antibody" as used herein may refer to a protein comprising one or more polypeptides (comprising one or more binding domains, preferably antigen binding domains) substantially or partially encoded by immunoglobulin genes or fragments of immunoglobulin genes. The term "immunoglobulin" (Ig) is used interchangeably with "antibody" herein. The recognized immunoglobulin genes include the kappa, lambda, alpha, gamma, delta, epsilon and mu constant region genes, as well as myriad immunoglobulin variable region genes. In particular, an "antibody" when used herein, is typically tetrameric glycosylated proteins composed of two light (L) chains of approximately 25 kDa each and two heavy (H) chains of approximately 50 kDa each. Two types of light chain, termed lambda and kappa, may be found in antibodies. Depending on the amino acid sequence of the constant domain of heavy chains, immunoglobulins can be assigned to five major classes: A, D, E, G, and M, and several of these may be further divided into subclasses (isotypes), e.g., IgG1, IgG2, IgG3, IgG4, IgA1, and IgA2, with IgG being preferred in the context of the present invention. An antibody of the present invention is also envisaged which has an IgE constant domain or portion thereof that is bound by the Fc epsilon receptor I. An IgM antibody consists of 5 of the basic heterotetramer unit along with an additional polypeptide called a J chain, and contains 10 antigen binding sites, while IgA antibodies comprise from 2-5 of the basic 4-chain units which can polymerize to form polyvalent assemblages in combination with the J chain. In the case of IgGs, the 4-chain unit is generally about 150,000 Daltons. Each light chain includes an N-terminal variable (V) domain (VL) and a constant (C) domain (CL). Each heavy chain includes an N-terminal V domain (VH), three or four C domains (CHs), and a hinge region. The constant domains are not involved directly in binding an antibody to an antigen, but can exhibit various effector functions, such as participation of the antibody dependent cellular cytotoxicity (ADCC). If an antibody should exert ADCC, it is preferably of the IgG1 subtype, while the IgG4 subtype would not have the capability to exert ADCC.

The term "antibody" also includes, but is not limited to, but encompasses monoclonal, monospecific, poly- or multi-specific antibodies such as bispecific antibodies, humanized, camelized, human, single-chain, chimeric, synthetic, recombinant, hybrid, mutated, grafted, and in vitro generated antibodies, with chimeric or humanized antibodies being preferred. The term "humanized antibody" is commonly defined for an antibody in which the specificity encoding CDRs of HC and LC have been transferred to an appropriate human variable frameworks ("CDR grafting"). The term "antibody" also includes scFvs, single chain antibodies, diabodies or tetrabodies, domain antibodies (dAbs) and nanobodies. In terms of the present invention, the term "antibody" shall also comprise bi-, tri- or multimeric or bi-, tri- or multifunctional antibodies having several antigen binding sites.

Furthermore, the term "antibody" as employed in the context of the present invention also relates to derivatives of the antibodies (including fragments) described herein. A "derivative" of an antibody comprises an amino acid sequence which has been altered by the introduction of amino acid residue substitutions, deletions or additions. Additionally, a derivative encompasses antibodies which have been modified by a covalent attachment of a molecule of any type to the antibody or protein. Examples of such molecules include sugars, PEG, hydroxyl-, ethoxy-, carboxy- or amine-groups but are not limited to these. In effect the covalent modifications of the antibodies lead to the glycosylation, pegylation, acetylation, phosphorylation, amidation, without being limited to these.

As used herein the term "antigen binding portion" refers to a fragment of immunoglobulin (or intact antibody), and encompasses any polypeptide comprising an antigen-binding fragment or an antigen-binding domain. Preferably, the fragment such as Fab, F(ab'), F(ab')₂, Fv, scFv, Fd, disulfide-linked Fvs (sdFv), and other antibody fragments that retain antigen-binding function as described herein. Typically, such fragments would comprise an antigen-binding domain and have the same properties as the antibodies described herein.

The terms "antigen-binding domain", "antigen binding portion", "antigen-binding fragment" and "antibody binding region" when used herein may refer to a part of an antibody molecule that comprises amino acids responsible for the specific binding between antibody and antigen. The part of the antigen that is specifically recognized and bound by the antibody is referred to as the "epitope" as described herein. As mentioned above, an antigen-binding domain may typically comprise an antibody light chain variable region (VL) and an antibody heavy chain variable region (VH); however, it does not have to comprise both. Fd fragments, for example, have two VH regions and often retain some antigen-binding function of the intact antigen-binding domain.

The term "antigen" as used herein may refer to a molecule or substance which induces an immune response (preferably an antibody response) in an animal, preferably a non-human animal immunized therewith (i.e. the antigen is "immunogenic" in the animal).

In the context of the present invention, the term "protein" may also comprise fusion proteins. The term "fusion protein" may refer to a genetically engineered protein that is created by joining two or more individual protein coding sequences.

As used herein, the terms "nucleic acids" or "nucleotide sequences" may refer to DNA molecules (e.g. cDNA or genomic DNA), RNA (mRNA), combinations thereof or hybrid molecules comprised of DNA and RNA. The nucleic acids can be double- or single-stranded and may contain double- and single-stranded fragments at the same time.

Smaller biomolecules that are envisaged to be introduced to a subject using the implant of the present invention are for example vitamins, hormones and signaling molecules.

Chemical molecules are e.g. chemotherapeutics. Chemotherapeutic may comprise alkylating agents, such as nitrogen mustard, nitrosourea, platinum analogs, thiazine or alkyl sulfonate, anti-metabolites, such as cytidine analogs, folate antagonists, purine analogs or pyrimidine analogs, anti-microtubular agents, such as Topoisomerase II inhibitors, Topoisomerase I inhibitors, taxanes or vinca alkaloids, antibiotics, such as MOA or Bleomycin, and miscellaneous, such as hydroxyurea, tretinoin, arsenic trioxide or proteasome inhibitors, as described e.g. in Amjad et al. 2023, "Cancer Chemotherapy", StatPearls [Internet], last update February 27, 2023).

As explained above, in the context of the medical implant of the present invention, it envisaged that the molecule is adsorbed to and desorbed from the stationary phase in a electrically controllable manner. Accordingly, the molecule is at least temporarily immobilized on the stationary phase. The terms "immobilized" or "immobilization" as used herein may refer to the process of fixing or attaching a molecule, substance, or entity in a stable and relatively fixed position, onto a surface or within a matrix. This immobilization is typically achieved through chemical or physical means, and it results in the molecule or substance becoming effectively stationary or restricted in its movement.

Within the context of the present invention it is particularly envisaged that said stationary phase is capable of adsorbing said molecules through a ligand immobilized on said stationary phase. The term "immobilized on a stationary phase" as used herein may mean that the ligand is firmly attached or affixed to the solid or semi-solid medium (i.e., the stationary phase) through which the mobile phase passes. Moreover, as set forth herein above, it is also envisaged that the molecule is adsorbed to the stationary phase before the stationary phase if placed between the electrodes inside the device of the implant.

Immobilization can be achieved through various mechanisms, for example, including but not limited to: (1) Physical adsorption, where molecules or chemical entities adhere to the surface of the stationary phase because of weak forces like van der Waals interactions; (2) Covalent bonding, where molecules or chemical entities are chemically bound to the surface, often through linker molecules or specific functional groups on the surface; (3) Entrapment, where molecules or chemical entities are trapped in a network or matrix, but not directly bound to it; (4) Ionic interactions, often used in ion-exchange chromatography, where charged species or chemical entities are retained on a stationary phase with an opposite.

Said ligands immobilized on the stationary phase may also be referred to as "immobilization sites" of the molecules. The immobilization sites/ligands are preferably affixed to either the inner or outer surface of a (exemplary porous) stationary phase. In some aspects, the inner surface refers to a surface within the open pores of the stationary phase. These inner surfaces are preferably accessible to external fluids. In some embodiments, the stationary phase is predominantly constructed from a porous substrate, e.g., a membrane, preferably composed of polymer/s. The immobilization sites of the molecules or chemical entities can be on either the inner or outer surface of this porous substrate. In some aspects, immobilization of chemical entities comprise, among others, 1-Ethyl-3-(3-dimethylaminopropyl)carbodiimid (EDC) or Dicyclohexylcarbodiimid (DCC) coupling and/or radical coupling methods. Specifically, these also include methods like electronic beam treatment.

In some aspects, utilizing EDC or DCC couplling for immobilization of e.g. ligands that create the binding sites for the molecules to be purified from the liquid generally results in a reasonably uniform orientation of these binding sites on the surface(s) of said stationary phase (see **Figure** 2). However, the yield of binding is often relatively low. Notably, employing EDC or DCC requires an initial grafting step to prepare the surface(s) of the stationary phase(s) for immobilization. This preparation involves generating amine- or carboxy- moieties on these surfaces, which then serve as sites for subsequent immobilization of chemical entities using processes like amination or similar methods. Conversely, the use of radical coupling techniques presents a less extensive approach for immobilizing binding sites onto the stationary phase(s). Typically, stationary phases that incorporate binding sites generated through radical coupling methods exhibit greater quantities of binding sites. Nonetheless, these binding sites might exhibit a less uniform arrangement or orientation.

Within the setup of the medical implant of the present invention, it is envisaged that the ligand immobilized on the stationary phase and representing the binding sites for the molecules is capable of non-covalently binding to said molecules, i.e. the binding between the ligand and the molecules is preferably a non-covalent binding. The terms "non-covalent binding" or "non-covalently bind" or "non-covalently bound" as used herein can be used interchangeably and do not involve the permanent sharing of electrons by way of a chemical bond. It involves variations of electromagnetic interactions between molecules. Examples of electromagnetic interactions are ionic interactions, hydrogen bonding, halogen bonding, van der Waals interaction, dipole-dipole interactions, dipole-induced dipole interaction, London dispersion interaction. In the context of the present invention it is envisaged that the molecules are known to non-covalently bind to the ligand immobilized on the stationary phase. In particular, it is envisaged that said ligand is capable of non-covalently binding said molecules when the voltage is changed between said electrodes as described elsewhere herein. In this respect the used molecules are known to non-covalently binding to the ligand immobilized on the stationary phase.

Exemplarily ligands that may be immobilized to the stationary phase and can be used for non-covalently binding biomolecules, such as proteins, are protein affinity tags, such as the E2 epitope, FLAG epitope, EE-tag, HA tag, HSV epitope, KT3 epitope, Myc epitope, S1-tag, T7 epitope, VSV-G, V5-tag. Further, protein A (e.g., having Uniprot Accession Number: P02976), protein G (e.g., having Uniprot Accession Number: P06654) and/or protein L (e.g., having Uniprot Accession Number: Q53291) can be used as ligands to e.g. non-covalently binding antibodies. The term "protein A" as used herein may refer to a surface protein derived from the bacterium *Staphylococcus aureus.* It has the unique ability to bind to the constant region (Fc region) of immunoglobulins, particularly immunoglobulin G (IgG), from various species. Protein A (e.g., having Uniprot Accession Number: P02976) is commonly used in affinity chromatography to purify antibodies. The interaction between protein A and antibodies can also be exploited for antibody detection, immunoprecipitation, and/or other applications. Additionally, recombinant forms of protein A are engineered to optimize binding efficiency and specificity, enhancing its utility in various processes. The term "protein G" as used herein may refer to bacterial surface protein originally isolated from certain strains of bacteria, primarily of the species *Streptococcus.* Similar to protein A, protein G possesses the ability to bind to the Fc region of immunoglobulins (IgG antibodies), making it a valuable tool for purification. Protein G (e.g., having Uniprot Accession Number: P06654) can be used in affinity chromatography to purify antibodies from complex samples. When immobilized on a solid support matrix, protein G columns can selectively capture and purify IgG antibodies. This process takes advantage of the specific interaction between protein G and the Fc region of antibodies. While protein A has a higher affinity for IgG antibodies from certain species (such as human, rabbit, and pig), protein G offers broader species reactivity, making it particularly useful for purifying antibodies from a wider range of sources, including mouse, rat, and bovine. Recombinant forms of protein G have also been engineered to optimize its binding properties and enhance its effectiveness in various processes. The term "protein L" as used herein may refer to a bacterial surface protein derived from certain strains of bacteria, such as *Peptostreptococcus magnus.* Protein L is unique in its ability to bind to the light chain of immunoglobulins (Ig) from various species, regardless of the species' heavy-chain subclass. This characteristic sets protein L (e.g., having Uniprot Accession Number: Q53291) apart from other antibody-binding proteins like protein A and protein G, which primarily bind to the Fc region of IgG antibodies. Due to its ability to interact with the variable region of immunoglobulin light chains, protein L is particularly useful for the purification and detection of antibodies that might not bind well to protein A or protein G, such as camelid antibodies (e.g., llama, alpaca) that lack a traditional Fc region. Recombinant forms of protein L have also been engineered to optimize its binding properties and enhance its effectiveness in various processes. In this respect the provided means can be particularly utilized for a potential-controlled affinity membrane chromatography for antibody purification.

In the context of the present invention said ligands immobilized to the stationary phase may also comprise peptide and nuclei acid aptamers. The term "aptamer" as used herein may refer to short sequences of artificial DNA, RNA, XNA, or peptide that bind a specific target molecule, or family of target molecules. Aptamers can be selected (e.g., through SELEX (Systematic Evolution of Ligands by Exponential Enrichment)) for their ability to bind to a specific target molecule with high affinity and specificity. Typically aptamers can selectively recognize and bind to a wide range of target molecules, including proteins, nucleic acids, small molecules, and even cells.

The term "peptide aptamer" as used herein may refer to a short peptide sequence that binds a specific target molecule, or family of target molecules. Peptide aptamers can be selected (e.g., through SELEX (Systematic Evolution of Ligands by Exponential Enrichment)) for their ability to bind to a specific target molecule with high affinity and specificity. Peptide aptamer is preferably based on a scaffold, e.g. thioredoxin, adnectin, anticalin, avimer, knottin, fynomer, atrimer, darpin, affibody, affilin, armadillo repeat, Obody (e.g., Reyerdatto et al. (2015), doi: 10.2174/1568026615666150413153143).

The term "DNA aptamer" as used herein may refer to short single-stranded DNA molecule that binds a specific target molecule, or family of target molecules. DNA aptamers can be selected (e.g., through SELEX (Systematic Evolution of Ligands by Exponential Enrichment)) for their ability to bind to a specific target molecule with high affinity and specificity.

The term "RNA aptamer" as used herein may refer to a short single-stranded RNA molecule that binds a specific target molecule, or family of target molecules. RNA aptamers can be selected (e.g., through SELEX (Systematic Evolution of Ligands by Exponential Enrichment)) for their ability to bind to a specific target molecule with high affinity and specificity.

In the context of the present inventions also ligands such as protein-binding proteins or carbohydrate-binding domains can be used. Exemplarily pairs of a ligand immobilized to the stationary phase and the corresponding molecules in the liquid binding to said ligand are selected from, e.g. an antigen and its corresponding antibody (preferably a monoclonal antibody), albumin (e.g., having Uniprot Accession Number: P02768) and albumin-binding protein (ABP), avidin (e.g., having Uniprot Accession Number: P02701) and biotin-carboxy carrier protein (BCCP) (e.g., having Uniprot Accession Number: P0ABD8), streptavidin (e.g., having Uniprot Accession Number: P22629) and biotin-carboxy carrier protein (BCCP) (e.g., having Uniprot Accession Number: P0ABD8), calmodulin (e.g., having Uniprot Accession Number: P62152) and calmodulin binding peptide (CBP), chloramphenicol (e.g., CAS Registry Number: 56-75-7) and chloramphenicol acetyl transferase (CAT) (e.g., having Uniprot Accession Number: P11504), cellulose and cellulose binding domain (CBP), chitin and chitin binding domain (CBD), choline and choline-binding domain (CBD), galactose and galactose-binding protein (GBP) (e.g., having Uniprot Accession Number: P0AEE5), glutathione and glutathione S-transferase (GST) (e.g., having Uniprot Accession Number: P08515), divalent metal ion, such as Ni2+, Co2+, Cu2+ or Zn2+ and histidine affinity tag (HAT), divalent metal ion, such as Ni2+, Co2+, Cu2+ or Zn2+ and poly-histidine (His-tag), cross-linked amylose or maltose and maltose-binding protein (MBP) (e.g., having Uniprot Accession Number: P0AEX9), streptavidin (e.g., having Uniprot Accession Number: P22629) and streptavidin binding peptide (SBP), Strep-Tactin and Strep-tag, protein A and antibody (in particular lgG1, IgG2 or IgG4), protein G and antibody (in particular lgG1, IgG2, IgG3 or IgG4), protein L and antibody (in particular, IgG, IgA, IgM, IgD, IgE), strep-tactin and strep-tag (e.g., Kimple et al. (2013), doi: 10.1002/0471140864.ps0909s73), but the invention is not limited thereto.

The medical implant of the present invention may be configured to comprise a stationary phase (or more than one stationary phase) varying regarding size, shape or material as long as the aforementioned functionalities may be satisfied. In particular, in some embodiments the implant of the present invention is configure the way that the stationary phase comprises at least two stationary phases. In further embodiments, the at least two stationary phases (e.g., distinct), including all of them if applicable, along with the affinity substrates, particularly membranes, and the specific affinity substrates, also membranes, or the respective non-conductive carriers that constitute these stationary phases, can be structured in various arrangements. These arrangements might include stacked, wrapped or tortuous arrangement, or being positioned separately in a disjunct pattern. Thus, in some embodiments the at least two stationary phases are stacked. In some embodiments the at least two stationary phases are wrapped. In some embodiments the at least two stationary phases are tortuous. In some embodiments the at least two stationary phases are located between two electrodes. In some embodiments, each of the two stationary phases is located between a separate set of two electrodes. Specifically, the distinct stationary phases may be positioned apart from one another along the direction of fluid flow containing the (bio)molecules.

In some aspects, in a stacked configuration, the at least two distinct, specifically different specific stationary phases, along with the affinity substrates, particularly membranes, and the specific affinity substrates, also membranes, or the respective non-conductive carriers, are organized as successive layers, forming a cohesive stack. This stack is ideally composed of at least two porous substrates, e.g., permeable membranes, that are aligned as stacked layers. More specifically, the stack is structured such that one of the electrodes, potentially formed by a conductive material like a metallic coating or metallic net and placed on a first affinity substrate, or membrane, and/or first non-conductive carrier, is positioned at one end of the stack. Correspondingly, the second electrode, also potentially formed by a conductive material and located on a second affinity substrate, or membrane, and/or second non-conductive carrier, is positioned at the opposite end of the stack. The stack encompasses an affinity substrate, particularly a membrane, core positioned between these first and second electrodes. This core comprises at least one affinity substrate, especially a membrane, and/or specific affinity substrates, also membranes, serving as the insulating barrier between the two electrodes.

In some aspects, electrodes can be positioned between each distinct stationary phase and/or amidst the layers, particularly affinity substrates or membranes, forming the stationary phases. Similarly, electrodes can also be positioned between layers of specific affinity substrates, or membranes, within the distinct stationary phases. Additionally, electrodes can be placed between layers, stationary phases, or substrates, especially membranes, forming sub-cores. These sub-cores could include their respective ligands and/or receptors.

In some aspects, the at least two distinct stationary phases, preferably specific ones, are ideally positioned sequentially along the direction of fluid flow, particularly the flow of the first fluid. This arrangement involves aligning these stationary phases in a consecutive manner or one after the other. Alternatively, the first fluid can be directed in sequence across these at least two specific stationary phases. In this context, it is preferred that the two stationary phases represent different specific configurations.

In further aspects, a configuration involving the wrapped or tortuous of stationary phases, specifically constructed from substrates like membranes, particularly layers of stacked membranes incorporating at least two or all electrodes, is contemplated. In this arrangement, a stack of stationary phases and electrodes is preferably combined through wrapping or twisting, winding, or curving in a complex or convoluted manner. This kind of arrangement holds significant utility within chromatographic methods, including but not limited to the methods described herein. Such a configuration could effectively function as an integral component of various chromatographic apparatuses suitable for these purposes.

In some aspects, the creation of the wrapped arrangement involves folding a stationary phase or, alternatively, a stack of stationary phases (e.g., membranes), preferably encompassing at least two or all electrodes, into multiple wraps. In the case of the tortuous arrangement, the stationary phase or stack of stationary phases is preferably arranged in a series of twisted, wound, or curved in a complex or convoluted manner layers. This wrapped or tortuous configuration is subsequently positioned within a housing.

In further aspects, the chosen housing of a device can accommodate the wrapped or tortuous configuration in a manner that permits fluids to pass over and/or through the stationary phase(s) (e.g., membranes). The housing is designed to enable a specified flow direction, typically facilitated by at least one inlet for introducing fluids and at least one outlet for releasing them as described elsewhere herein. Within this housing, the wrapped or tortuous arrangement is positioned to obstruct direct passage from the inlet to the outlet. Furthermore, it ensures that any fluid flow from the inlet to the outlet necessitates passing through and/or over the stationary phase(s). This interaction typically occurs at least once, preferably multiple times, and can span various sections or regions of the stack and/or substrates. This encompasses traversal over and/or through all distinct specific phases within the arrangement. The wrapped or tortuous configuration is usually positioned within the housing in such a way that its main extension is oriented parallel, perpendicular, or oblique to the aforementioned designated flow direction.

In some aspects, cylindrical structures resembling tubes can serve as enclosures for configurations of the present invention. These tube-like structures may exhibit various base shapes, which can range from rectangular to circular, and include other forms. Additionally, suitable enclosures can take on a box-like structure, equipped with inlets and outlets placed between which the stationary phase(s) are positioned. These stationary phase(s) placed between the inlets and outlets can receive lateral fluid inputs. In this arrangement, the stationary phase(s) are positioned to obstruct direct passage from inlet to outlet. Fluid being channeled through the outlets is therefore compelled to traverse through and, preferably, over the stationary phase(s) at least once. Consequently, the inlets need to be situated on one side of the stationary phase(s), while the outlets are positioned on the opposite side, ideally diagonally opposite, of the stationary phase(s). This arrangement promotes the desired fluid flow dynamics.

In further aspects, circular enclosures characterized by an inner core and an outer cage, separated by a gap, can be used as housing. The gap, defined by the shortest distance between the core and the cage, serves as a crucial element in this context. Within this gap, the wrapped or tortuous surface is positioned. Both the inner core and the outer cage, in an ideal scenario, exhibit permeability to fluids and might also include inlets and outlets for fluid supply to the surface(s) or for the removal of fluids/eluate. The fluid, for optimal flow, is expected to pass through the arrangement radially within the gap, facilitating its flow from the inlet to the outlet. Typically, only either the inner core or the outer cage is equipped with inlets, while the other component has outlets. An inlet could be any form of fluid-permeable passage facilitating supply, and an outlet refers to a passage for removal. For example, the volume within the core can be utilized for either the introduction of fluids or the removal of fluids.

In some aspects, the wrapped or tortuous configuration comprises a minimum of two porous substrates, preferably made from polymeric membranes. On one side of each respective membrane, a conductive coating or layer of metal is applied, forming a stacked assembly. Additionally, non-conductive porous substrates, ideally made from polymeric membranes, are interposed between the coated membranes. This arrangement establishes at least one stationary phase within the system.

In some aspects, an insulator (e.g., made from one of the porous substrates, e.g., polymeric membranes), is positioned amidst the electrically conductive coatings or layers. This configuration can be harnessed to intentionally administer release voltages exclusively to chosen substrates.

In some aspects, the ratio of surface area to volume can be influenced by the choice of stationary phase(s) arrangement, particularly concerning the housing structure. In this context, the wraps are comprised of flat sections positioned between local folding points. Each of these flat areas has a length, and the preferred form for the pleats is an M-shape. For every wrap, a first length is selected to exceed at least one dimension such as width, diameter, or similar attributes of the housing. Specifically, for circular housings with an outer cage and inner core, this pertains to the gap distance. Considering that the core has a smaller circumference than the cage, wraps with uniform, nearly identical lengths of surface areas would lead to suboptimal surface area to volume ratios. Hence, wraps with irregular M-shapes are more desirable as they yield higher, optimally optimized surface area to volume ratios. These irregular M-shapes consist of flat areas with at least one second length that differs from the first length. Preferably, sets of wraps encompassing more than one wrap are used. These wrap sets can include wraps characterized by at least two distinct irregular M-shapes. These sets of wraps are preferably repeated in a regular manner within the housing. Particularly in the context of circular housing, the wraps or wraps sets can create a complete circle within the housing. Regarding tortuous arrangements, circular housings can also offer advantages. The winding can be achieved by enveloping the surface(s) or stack of surface(s) around the inner core of the housing, with the inner core playing a crucial role in supporting the wrapped structure.

In some further aspects, the present invention relates to a medical implant comprising fewer membranes stacked on top of each other (e.g., compared to Sartobind Protein A75) enabling a much faster loading (e.g., adsorption) of the membranes with antibodies. The potential-controlled rapid discharge (e.g., desorption) of the membrane and/or immediate rechargeability according to the present invention results in a better performance.

The medical implant of the present invention is particularly envisaged to be used to treating a medical condition or a disease in a subject. Accordingly, the present invention also provides for a molecule adsorbed to the stationary phase of the implant described herein for use in a method of treating a medical condition with said molecule, said treatment comprising desorbing said molecule from said stationary phase of said implant implanted into said subject by changing the voltage between said electrodes of said implant. Similarly, the present invention also refers to a method of treatment of a medical condition of a subject in need of a treatment with a ligand adsorbed to the stationary phase of the implant of any one of the preceding claims, comprising desorbing said molecule from said stationary phase of said implant implanted into said subject by changing the voltage between said electrodes of said implant.

The terms "treat", "treating" or "treatment" as used herein in respect to a disease or a medical condition generally refer to obtaining a desired pharmacologic and/or physiologic effect in a subject. The effect may be prophylactic in terms of completely or partially preventing a disease or symptom thereof and/or may be therapeutic in terms of a partial or complete stabilization or cure for a disease and/or adverse effect attributable to the disease. "Treatment" as used herein covers any treatment of a disease in a mammal, particularly a human, and includes: (a) preventing the disease or symptom from occurring in a subject which may be predisposed to the disease or symptom but has not yet been diagnosed as having it; (b) inhibiting the disease symptom, i.e., arresting its development; or (c) relieving the disease symptom, i.e., causing regression of the disease or symptom. Said subject is expected to have undergone physical examination by a medical or veterinary medical practitioner, who has given a tentative or definitive diagnosis which would indicate that the use of the molecule(s) introduced into said subject for treatment of a disease or condition is beneficial to the health of said human or other animal subject. Thus, a " subject in need of a treatment" means that said subject - according to the medical practitioner's analysis and assessment - may benefit from said treatment. However, timing, doses and purpose of said treatment may vary from one individual to another, according to the status quo of the subject's health. Thus, said treatment may be palliative, symptomatic and/or curative.

The term "subject" as used herein, also addressed as an individual or patient, refers to a mammal organism, including a human or a non-human animal. Thus, means and methods described herein are generally applicable to both human and non-human mammals. A non-human animal may also represent a model of a particular disease or disorder. Alternatively, a non-human animal may represent a domesticated pet and/or livestock in need of treatment of a disease (for example a dog, cat, goat, bovine, ovine, etc.). Preferably, the subject of the present invention is a mammalian subject, in particular a human, a non-human primate, a dog, a horse, a cat, a guinea pig, a rabbit, a rat or a mouse.

When using the molecules adsorbed to the stationary of the implant for the treatment of a medical condition, it is envisaged that the molecule is desorbed from the stationary phase of the implant and introduced to the subject in a therapeutically effective dose. Hence, as described elsewhere herein, the implant is preferably pre-loaded with the molecules to be desorbed from the stationary phase before placing the implant on or in a subject. By "therapeutically effective dose", "therapeutically effective amount" or "effective amount" of a compound used for treating a medical condition is intended an amount of the molecule that, when administered to the subject brings about a positive therapeutic response with respect to treatment of the respective condition. As is known in the art, such a therapeutically effective dose depends on the age, body weight, general health, sex, diet, drug interaction and the severity of the condition to be treated, and will be ascertainable with routine experimentation. The therapeutic effect when using the means provided by the present invention may be detectable by established methods and approaches which will indicate a therapeutic effect. For example, it is envisaged that the therapeutic effect is detected by way of surgical resection or biopsy of an affected tissue/organ which is subsequently analyzed. Alternatively, e.g. when treating cancer, it is also envisaged that the tumor markers in the serum of the subject (if present) are detected in order to diagnose whether the therapeutic approach is already effective or not. Additionally or alternatively it is also possible to evaluate the general appearance of the respective patient (fitness, well-being, decrease of tumor-mediated ailment etc.) which will also aid the skilled practitioner to evaluate whether a therapeutic effect is already there. The skilled person is aware of numerous other ways which will enable him or her to observe a therapeutic effect of a molecule or compounds when administered to a subject utilizing the medical implant provided by the present invention.

The terms "disease", "disorder" or "(medical) condition" as used herein refers to any impairment of the normal state of a living human or animal subject or one of its parts that interrupts or modifies the performance of vital functions that is typically manifested by distinguishing signs and symptoms. Thus, a "disease", "disorder" or "condition" refers to any physical state of a subject connected with incorrectly functioning organ, part, structure, or system of the body resulting from the effect of genetic or developmental errors, infection, poisons, nutritional deficiency or imbalance, toxicity, or unfavorable environmental factors, illness, sickness, or ailment. Such medical condition may e.g. be a cancerous disease, neurodegenerative diseases, vascular diseases, inflammatory disease, or diabetes, just to name some. Further, the medical implant may be used for pain management, such treatment of chronic pain.

The term, "cancerous disease" refers to a disorders related to the development of abnormal cells that divide uncontrollably and have the ability to infiltrate and destroy normal body tissue. Cancer is a large group of diseases and comprises inter alia cancer of the skin, breast, lung, liver, pancreas, colon, stomach, prostate, blood, bone and the nervous system.

The term "inflammatory disease" as used herein refers to the complex biological response of body tissues to harmful stimuli, such as pathogens, damaged cells, or irritants, and is a protective response involving immune cells, blood vessels, and molecular mediators. Inflammatory diseases comprise e.g. allergy, asthma, psoriasis, autoimmune disease, rheumatoid arthritis, coeliac disease, glomerulonephritis, hepatitis, inflammatory bowel disease, reperfusion injury, and transplant rejection.

"Neurogenerative diseases" are *chronic conditions that damage and destroy parts of the* nervous system over time, especially the brain. Neurogenerative disease comprise e.g. Alzheimer's disease and other memory disorders, ataxia, Huntington's disease, Parkinson's disease, motor neuron disease, multiple system atrophy, and progressive supranuclear palsy.

The term "vascular disease" as used herein refers to diseases of the vessels of the circulatory system in the body, including blood vessels - the arteries and veins, and the lymphatic vessels. Vascular disease comprise e.g. aneurysm, carotid artery disease, deep vein thrombosis, mesenteric artery disease, peripheral artery disease, renal artery disease, vascular malformations, varicose, and spider veins.

The term "diabetes" as used herein refers to diabetes mellitus, i.e. a chronic, metabolic disease characterized by elevated levels of blood glucose (or blood sugar), which leads over time to serious damage to the heart, blood vessels, eyes, kidneys and nerves. The most common is type 2 diabetes, usually in adults, which occurs when the body becomes resistant to insulin or doesn't make enough insulin. Type 1 diabetes, once known as juvenile diabetes or insulin-dependent diabetes, is a chronic condition in which the pancreas produces little or no insulin by itself. Known medicaments for treating diabetes are e.g. metformin, sulfonylureas, DPP-4-inhibitors, gliflozines, glutides and insulin.

When treating a subject by using the implant of the present invention it is envisaged that said implant is permanently or temporarily placed on the surface or inside the body of the subject to allow desorption of the molecule(s) from the stationary phase of said implant by changing the voltage between the electrodes of said implant and to deliver medication to said subject. In this respect it is particularly envisaged that the molecule to be delivered to the subject is already adsorbed to the stationary phase of the implant when the implanted is placed on or in the subject. However, it is also within the context of the therapeutic application of the implant of the present invention that the implanted product can be removed again from the subject after a first treatment cycle and reloaded with the active ingredient(s). Thus, multiple/repeated uses of the implant, i.e. repeated adsorption and desorption of the molecule to and from the stationary phase of the implant, are possible. Alternatively, the implanted product may be re-loaded when still implanted on or in the subject, e.g. using external means like a syringe or pump.

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "less than" or in turn "more than" does not include the concrete number.

For example, less than 20 means less than the number indicated. Similarly, more than or greater than means more than or greater than the indicated number, e.g., more than 80 % means more than or greater than the indicated number of 80 %.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. When used herein the term "comprising" can be substituted with the term "containing" or "including" or sometimes when used herein with the term "having". When used herein "consisting of" excludes any element, step, or ingredient not specified.

The term "including" means "including but not limited to". "including" and "including but not limited to" are used interchangeably.

One skilled in the art would readily appreciate that the present invention is well adapted to carry out the objects and obtain the ends and advantages mentioned, as well as those inherent therein. Further, it will be readily apparent to one skilled in the art that varying substitutions and modifications may be made to the invention disclosed herein without departing from the scope and spirit of the invention. The compositions, methods, procedures, treatments, molecules and specific compounds described herein are presently representative of certain embodiments are exemplary and are not intended as limitations on the scope of the invention. Changes therein and other uses will occur to those skilled in the art which are encompassed within the spirit of the invention are defined by the scope of the claims. The listing or discussion of a previously published document in this specification should not necessarily be taken as an acknowledgement that the document is part of the state of the art or is common general knowledge.

The invention illustratively described herein may suitably be practiced in the absence of any element or elements, limitation or limitations, not specifically disclosed herein. Thus, for example, the terms "comprising", "including," containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by exemplary embodiments and optional features, modification and variation of the inventions embodied herein may be resorted to by those skilled in the art, and that such modifications and variations are considered to be within the scope of this invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein. All documents, including patent applications and scientific publications, referred to herein are incorporated herein by reference for all purposes.

Other embodiments are within the following claims. In addition, where features or aspects of the invention are described in terms of Markush groups, those skilled in the art will recognize that the invention is also thereby described in terms of any individual member or subgroup of members of the Markush group.

It should be understood that this invention is not limited to the particular methodology, protocols, material, reagents, and substances, etc., described herein and as such can vary. The terminology used herein is for the purpose of describing particular embodiments only and is not intended to limit the scope of the present invention, which is defined solely by the claims.

All publications cited throughout the text of this specification (including all patents, patent application, scientific publications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

The content of all documents and patent documents cited herein is incorporated by reference in their entirety.

The present invention is further characterized by the following items:
1. A medical implant comprising in its interior a device for electrically controllable ad- and desorption of molecules, said device comprising:
   (a) two electrodes comprising a conductive surface,
   (b) a stationary phase between said two electrodes, wherein said stationary phase is not in direct contact with said electrodes,
   (c) a controller functionally associated with a power supply configured for applying and changing a voltage between said electrodes,
   wherein changing the voltage between said electrodes changes the affinity of molecules so as to adsorb said molecules to said stationary phase or desorb molecules from said stationary phase when said molecules are adsorbed to said stationary phase.
2. The implant of item 1, wherein said electrodes form a capacitor.
3. The implant of any one of the preceding items, wherein said stationary phase acts as a dielectric or insulator between the electrodes.
4. The implant of any one of the preceding items, wherein each of said electrodes comprises an insulating layer between said electrode and said stationary phase.
5. The implant of any one of the preceding items, wherein said electrodes are configured to allow flow of a liquid.
6. The implant of item 5, wherein said liquid is isotonic with blood.
7. The implant of item 5 or 6, wherein said flow of said liquid is in transverse direction and/or in longitudinal direction in relation to said stationary phase being located between said electrodes.
8. The implant of any one of the preceding items, wherein said voltage is DC voltage.
9. The implant of any one of the preceding items, wherein said voltage is from a constant voltage source, preferably an adjustable constant voltage source.
10. The implant of any one of the preceding items, wherein said controller is a voltage source which does not react on changing current.
11. The implant of any one of the preceding items, wherein said electrode is not formed by an electrically conductive coating and/or metallic net which is directly formed and/or disposed on said stationary phase.
12. The implant of any one of the preceding items, wherein said stationary phase is porous.
13. The implant of item 12, wherein said stationary phase is a membrane, a polymer, a non-metal material, felt or foam.
14. The implant of any one of the preceding items, wherein said stationary phase comprises at least two stationary phases.
15. The implant of item 14, wherein said at least two stationary phases are stacked, wrapped or tortuous.
16. The implant of item 14 or 15, wherein said at least two stationary phases are located between two electrodes.
17. The implant of item 14 or 15, wherein each stationary phase is located between a separate set of two electrodes.
18. The implant of any one of the preceding items, wherein said stationary phase is capable of adsorbing said molecules.
19. The implant of any one of the preceding items, wherein said stationary phase is capable of adsorbing said molecules when the voltage is changed between said electrodes.
20. The implant of any one of the preceding items, wherein said stationary phase is capable of adsorbing said molecules through a ligand immobilized on said stationary phase.
21. The implant of item 20, wherein said ligand is capable of non-covalently binding said molecules.
22. The implant of item 20 or 21, wherein said ligand is capable of non-covalently binding said molecules when the voltage is changed between said electrodes.
23. The implant of any one of the preceding items, wherein said stationary phase comprises molecules already adsorbed to said stationary phase or said ligand immobilized on said stationary phase.
24. The implant of any one of the preceding items, wherein said molecules adsorbed to said stationary phase are desorbed from said stationary phase or said ligands when the voltage is changed between said electrodes.
25. The implant of any one of the preceding items, wherein said molecules are biomolecules or chemical molecules.
26. The implant of item 25, wherein said biomolecule is a protein, preferably an antibody or insulin, or a nucleic acid, such as DNA or RNA.
27. The implant of item 25, wherein said chemical molecules are chemotherapeutics.
28. A molecule adsorbed to the stationary phase of the implant of any one of the preceding items for use in a treatment of a medical condition with said molecule, said method comprising desorbing said molecule from said stationary phase of said implant implanted into said subject by changing the voltage between said electrodes of said implant.
29. A method of treatment of a medical condition of a subject in need of a treatment with a molecule adsorbed to the stationary phase of the implant of any one of the preceding items, comprising desorbing said molecule from said stationary phase of said implant implanted into said subject by changing the voltage between said electrodes of said implant.

### EXAMPLES

An even better understanding of the present invention and of its advantages will be evident from the following examples, offered for illustrative purposes only. The examples are not intended to limit the scope of the present invention in any way.

### Example 1:

### Synthesis of iron oxide nanoparticles

The superparamagnetic iron oxide nanoparticles (IONs) were prepared by co-precipitation according to an already established and published protocol (Turrina et al. 2021, Pharmaceuticals 14, 405, doi:10.3390/ph140504051). To this end, 14.45 g (361.5 mmol) sodium hydroxide was dissolved in 200 mL degassed ultrapure water under nitrogen atmosphere. The solution was placed on a heating plate with a constant temperature of 27°C. 4 g (64 mmol) FeCl₃ (anhydrous) and 7 g (35.2 mmol) FeCl₂ • 4 H₂O were dissolved in 80 mL degassed ultrapure water. Once, the NaOH solution reached 27°C the iron-containing solution was added slowly to the solution and the reaction continued for 30 min at constant conditions. The obtained particles were transferred to a glass flask and washed 15 times with degassed ultrapure water by magnetic decantation until a pH 7 was reached. After that, the IONs were resuspended in 500 mL degassed ultrapure water and stored at 4°C. For the determination of the mass concentration, 10 mL ION suspension was transferred to three previously weighed 50 mL tubes, respectively, and the IONs were dried overnight at 60°C. The mass concentration was calculated by the difference in weight.

### Polymer coating of iron oxide nanoparticles

The polymer-coating of the IONs was performed based on two adapted and modified protocols (Yu and Chow 2004, J. Mater. Chem. 14, 2781-2786, doi:10.1039/B404964K; Mekseriwattana et al. 2019, AAPS PharmSciTech 20, 55, doi:10.1208/s12249-018-1275-x). 100 mg IONs were placed in a glass bottle with a ported cap and redispersed in 100 mL ultrapure water using an ultrasonic processor (Fisherbrand) with an energy of 75% amplitude. Afterwards, the suspension was placed in an ultrasound bath and connected to a vacuum source to degas for 30 min. The bottle was evacuated with nitrogen and further bubbled with nitrogen for 20 min. Under continuous stirring, 575 mg (2 mmol) sodium dodecyl sulfate was added and the suspension was heated to 70 °C in a water bath. Once the temperature reached 70 °C, the 205.9 µL (3 mmol) acrylic acid and 253.1 µL (3 mmol) methacrylic acid was added. After the addition of the monomers, the suspension was left to equilibrate for 45 min. Finally, 825 mg (3.61 mmol) ammonium persulfate was dissolved in 10 mL water added to the suspension to start the polymerisation. The reaction was left at a constant temperature of 70 °C. Throughout the entire synthesis, the head space of the bottle was purged with nitrogen. After 2 h, the poly(acrylic acid-co-methacrylic acid)-coated IONs [ION@P(AA-co-MAA)], were separated magnetically and washed with a total of 6 L of ultrapure water. The coated nanoparticles, were re-dispersed in ultrapure water and stored at 4 °C. For the determination of the mass concentration, 2 mL of the suspension was placed in a glass vial and dried in an oven at 60 °C.

### Experiments for drug release

A cover slip was fixed onto the O-ring of the cathode using nail polish and placed it in a small beaker. The beaker was filled with 6 mL of 25 mM phosphate buffer, pH 7.2, and immobilized ION@P(AA-co-MAA) was immobilized on this cover slip using a magnet. The particles were pre-loaded with LL-III and washed three times with buffer. A potential of 32 V was applied for 10 minutes, 20 minutes, and 30 minutes. After 10 minutes, the 6 mL of buffer were replaced with 6 mL of fresh buffer, and applied the potential for 20 minutes. The same procedure was followed for the 30-minute time point.

### Results

While no peptide has been released in this set-up just by washing the sample, an application of 32 V potential leads to a significant release of 16 µg drug after 30 minutes. This adds up to around 15% of the total amount of bound peptide in the system.

## Claims

1. A medical implant comprising in its interior a device for electrically controllable ad- and desorption of molecules, said device comprising:
(a) two electrodes comprising a conductive surface,
(b) a stationary phase between said two electrodes, wherein said stationary phase is not in direct contact with said electrodes,
(c) a controller functionally associated with a power supply configured for applying and changing a voltage between said electrodes,
wherein changing the voltage between said electrodes changes the affinity of molecules so as to adsorb said molecules to said stationary phase or desorb molecules from said stationary phase when said molecules are adsorbed to said stationary phase.

2. The implant of claim 1, wherein said electrodes form a capacitor, and/or wherein said stationary phase acts as a dielectric or insulator between the electrodes.

3. The implant of any one of the preceding claims, wherein each of said electrodes comprises an insulating layer between said electrode and said stationary phase.

4. The implant of any one of the preceding claims, wherein said electrodes are configured to allow flow of a liquid.

5. The implant of claim 5, wherein said liquid is isotonic with blood.

6. The implant of claim 5 or 6, wherein said flow of said liquid is in transverse direction and/or in longitudinal direction in relation to said stationary phase being located between said electrodes.

7. The implant of any one of the preceding claims, wherein said voltage is DC voltage, preferably wherein said voltage is from a constant voltage source, preferably an adjustable constant voltage source, preferably wherein said controller is a voltage source which does not react on changing current.

8. The implant of any one of the preceding claims, wherein said electrode is not formed by an electrically conductive coating and/or metallic net which is directly formed and/or disposed on said stationary phase.

9. The implant of any one of the preceding claims, wherein said stationary phase is porous, preferably wherein said stationary phase is a membrane, a polymer, a non-metal material, felt or foam, and/or wherein said stationary phase comprises at least two stationary phases, preferably wherein said at least two stationary phases are stacked, wrapped or tortuous and/or (a) wherein said at least two stationary phases are located between two electrodes or (b) wherein each stationary phase is located between a separate set of two electrodes.

10. The implant of any one of the preceding claims, wherein said stationary phase is capable of adsorbing said molecules, preferably wherein said stationary phase is capable of adsorbing said molecules when the voltage is changed between said electrodes.

11. The implant of any one of the preceding claims, wherein said stationary phase is capable of adsorbing said molecules through a ligand immobilized on said stationary phase, preferably wherein said ligand is capable of non-covalently binding said molecules, and/or wherein said ligand is capable of non-covalently binding said molecules when the voltage is changed between said electrodes.

12. The implant of any one of the preceding claims, wherein said stationary phase comprises molecules already adsorbed to said stationary phase or said ligand immobilized on said stationary phase.

13. The implant of any one of the preceding claims, wherein said molecules adsorbed to said stationary phase are desorbed from said stationary phase or said ligands when the voltage is changed between said electrodes.

14. The implant of any one of the preceding claims, wherein said molecules are biomolecules or chemical molecules, preferably wherein said biomolecule is a protein, preferably an antibody or insulin, or a nucleic acid, such as DNA or RNA, or wherein said chemical molecules are chemotherapeutics.

15. A molecule adsorbed to the stationary phase of the implant of any one of the preceding claims for use in a treatment of a medical condition with said molecule, said method comprising desorbing said molecule from said stationary phase of said implant implanted into said subject by changing the voltage between said electrodes of said implant.
